# EUROPEAN PATENT APPLICATION

(11) **EP 3 335 701 A1**
(43) Date of publication of application: **20.06.2018**
(21) Application number: 16204717.9
(22) Date of filing: 16.12.2016
(51) Int. Cl.: A61K 9/20, A61K 9/48, A61K 31/4162

(54) **PHARMACEUTICAL COMPOSITION COMPRISING OMARIGLIPTIN**

(71) Applicant: Hexal AG, 83607 Holzkirchen (DE)
(72) Inventor: BORN, Max, D-83607 Holzkirchen (DE); FISCHER-DAUT, Diana, D-83607 Holzkirchen (DE); PUZIK, Andreas, D-83607 Holzkirchen (DE)
(74) Representative: Kluschanzoff, Harald

(57) **Abstract**

The present invention relates to a pharmaceutical composition comprising omarigliptin or a pharmaceutically acceptable salt thereof and a diluent, wherein the diluent comprises a reducing sugar, a process for preparing the pharmaceutical composition and its use in the treatment of type 2 diabetes mellitus.

## Description

### FIELD OF THE INVENTION

The present invention relates to a pharmaceutical composition comprising omarigliptin or a pharmaceutically acceptable salt thereof and a diluent, wherein the diluent comprises a reducing sugar, a process for preparing the pharmaceutical composition and its use in the treatment of type 2 diabetes mellitus.

### BACKGROUND OF THE INVENTION

Omarigliptin (2R,3S,5R)-2-(2,5-difluorophenyl)-5-[2-(methylsulfonyl)-2,6-dihydropyrrolo[3,4-c]pyrazol-5(4H)-yl]tetrahydro-2H-pyran-3-amine acts as a dipeptidyl peptidase IV (DPP IV) inhibitor and is used for the treatment of type 2 diabetes mellitus.

WO 2010/056708 describes omarigliptin and a pharmaceutical composition comprising omarigliptin, microcrystalline cellulose, croscarmellose sodium, and magnesium stearate. Omarigliptin, microcrystalline cellulose and croscarmellose are blended first. The mixture is then lubricated by magnesium stearate and pressed into tablets. Stability of the formulation has not been tested.

WO 2015/031228 describes a stable pharmaceutical composition comprising omarigliptin and neutral excipients in an amount of at least 75% by weight of the pharmaceutical formulation, wherein the neutral excipients comprise at least one non-reducible sugar diluent. The diluent is mannitol or microcrystalline cellulose or a mixture of mannitol and microcrystalline cellulose. Exemplified pharmaceutical formulations are tablets and consist essentially of omarigliptin, mannitol, microcrystalline cellulose, croscarmellose sodium, and magnesium stearate. The tablets are prepared by wet or dry processing methods. Diluents that should not be included in the pharmaceutical formulations described in WO 2015/031228 include lactose monohydrate, polyvinyl pyrrolidone, silicified microcrystalline cellulose, hydroxyethyl cellulose, anhydrous lactose, mannose, glucose, maltose, other reducing sugars, Neusilin (aluminum magnesium metasilicate), Kollidon (polyvinyl pyrrolidone), crospovidone and those with acid functional groups or basic functional groups. It is disclosed that one concern with developing a pharmaceutical composition of omarigliptin is the chemical stability of the compound. Omarigliptin is said to be sensitive to oxidation, hydrolysis (acid and base catalysed) and Maillard degradation (browning) with reducible sugar impurities in excipients.

The pharmaceutical composition of WO 2015/031228 which contains two different diluents (mannitol and microcrystalline cellulose) has some disadvantages. When a pharmaceutical composition is manufactured on large scale, it is preferred to use a minimal number of excipients, for example, to reduce the number of analytical processes to release the ingredients, the number of containers used, the number of mixing steps, and the number of transfers of ingredients from one container to another. For large-scale industrial processes, the use of only one excipient instead of two excipients makes a significant difference with regard to complexity, productivity and cost efficiency.

### SUMMARY OF THE INVENTION

It has surprisingly been found that it is not necessary to use non-reducing excipients in pharmaceutical compositions containing omarigliptin to obtain a stable product. Other than suggested in WO 2015/031228, stable pharmaceutical compositions containing omarigliptin and the reducing sugar maltodextrin can be prepared.

Accordingly, the present invention provides a pharmaceutical composition comprising omarigliptin or a pharmaceutically acceptable salt thereof, and a diluent, wherein the diluent comprises a reducing sugar, and wherein the reducing sugar is maltodextrin.

By using maltodextrin as diluent instead of a mixture of mannitol and microcrystalline cellulose as in WO 2015/031228, no effect on the dissolution of the pharmaceutical composition is observed, no effect on the stability of the pharmaceutical composition is observed, but the efficiency of the manufacturing process, in particular on industrial scale, is considerably improved.

An additional advantage of the pharmaceutical composition containing a lower number of excipients is its potential smaller size. The size of a pharmaceutical composition, for example a tablet, has an important effect on patient compliance. In particular, in case of omarigliptin which is used in the treatment of type 2 diabetes, a disease prevalent amongst elderly people who may have to take several medicaments and may have swallowing problems, a smaller tablet size may increase patient compliance and, thus, treatment success.

### DETAILED DESCRIPTION OF THE INVENTION

The present invention provides a pharmaceutical composition comprising omarigliptin or a pharmaceutically acceptable salt thereof, and a diluent,
wherein the diluent comprises a reducing sugar, and
wherein the reducing sugar is maltodextrin.

Maltodextrin is a saccharide mixture of polymers that consist of D-glucose units connected in chains of variable length. Maltodextrin is prepared by partial hydrolysis of starch.

Maltodextrin is a reducible sugar, it gives a positive reaction in the Fehling test, i.e. maltodextrin reduces Fehling's solution.

Maltodextrin usually has a dextrose equivalent (DE) of 1 to 20. Preferably, maltodextrin having a dextrose equivalent of 3 to 20, more preferably maltodextrin having a dextrose equivalent of 5 to 10, and even more preferably maltodextrin having a dextrose equivalent of 3 to 5 is used. The dextrose equivalent is a measure of the extent of polymer hydrolysis and is expressed in grams of D-glucose per 100 g of the dry substance.

In a preferred embodiment of the present invention, the pharmaceutical composition comprises omarigliptin or a pharmaceutically acceptable salt thereof, and a diluent,
wherein the diluent comprises a reducing sugar,
wherein the reducing sugar is maltodextrin, and
wherein the maltodextrin is the only reducing sugar present in the pharmaceutical composition.

In another preferred embodiment of the present invention, the pharmaceutical composition comprises omarigliptin or a pharmaceutically acceptable salt thereof, and a diluent,
wherein the diluent comprises a reducing sugar,
wherein the reducing sugar is maltodextrin, and
wherein the maltodextrin is the only diluent present in the pharmaceutical composition.

In another preferred embodiment of the present invention, the pharmaceutical composition comprises omarigliptin or a pharmaceutically acceptable salt thereof, and a diluent,
wherein the diluent comprises a reducing sugar,
wherein the reducing sugar is maltodextrin, and
wherein the pharmaceutical composition is free of mannitol.

In another preferred embodiment of the present invention, the pharmaceutical composition comprises omarigliptin or a pharmaceutically acceptable salt thereof, and a diluent,
wherein the diluent comprises a reducing sugar,
wherein the reducing sugar is maltodextrin, and
wherein the pharmaceutical composition is free of microcrystalline cellulose.

In another preferred embodiment of the present invention, the pharmaceutical composition comprises omarigliptin or a pharmaceutically acceptable salt thereof, and a diluent,
wherein the diluent comprises a reducing sugar,
wherein the reducing sugar is maltodextrin, and
wherein the pharmaceutical composition is free of mannitol and free of microcrystalline cellulose.

In another preferred embodiment of the present invention, the pharmaceutical composition comprises maltodextrin in an amount of 60 to 99% by weight, preferably 60 to 90% by weight, more preferably 60 to 80% by weight, even more preferably 60 to less than 75% by weight, based on the pharmaceutical composition.

In another preferred embodiment of the present invention, the pharmaceutical composition comprises additionally a disintegrant and/or a lubricant.

An exemplified disintegrant is croscarmellose sodium.

An exemplified lubricant is magnesium stearate.

In another preferred embodiment of the present invention, the pharmaceutical composition comprises omarigliptin or a pharmaceutically acceptable salt thereof, a diluent, a disintegrant and a lubricant, wherein the diluent is maltodextrin, the disintegrant is croscarmellose sodium, and the lubricant is magnesium stearate.

In another preferred embodiment of the present invention, the pharmaceutical composition comprises omarigliptin or a pharmaceutically acceptable salt thereof, a diluent, a disintegrant and a lubricant, wherein the diluent is maltodextrin, the disintegrant is croscarmellose sodium, and the lubricant is magnesium stearate, and wherein maltodextrin is the only diluent present in the pharmaceutical composition.

In another preferred embodiment of the present invention, the pharmaceutical composition comprises omarigliptin or a pharmaceutically acceptable salt thereof, a diluent, a disintegrant and a lubricant, wherein the diluent is maltodextrin, the disintegrant is croscarmellose sodium, and the lubricant is magnesium stearate, and wherein the pharmaceutical composition does not comprise mannitol.

In another preferred embodiment of the present invention, the pharmaceutical composition comprises omarigliptin or a pharmaceutically acceptable salt thereof, a diluent, a disintegrant and a lubricant, wherein the diluent is maltodextrin, the disintegrant is croscarmellose sodium, and the lubricant is magnesium stearate, and wherein the pharmaceutical composition does not comprise microcrystalline cellulose.

In another preferred embodiment of the present invention, the pharmaceutical composition comprises omarigliptin or a pharmaceutically acceptable salt thereof, a diluent, a disintegrant and a lubricant, wherein the diluent is maltodextrin, the disintegrant is croscarmellose sodium, and the lubricant is magnesium stearate, wherein the pharmaceutical composition does not comprise mannitol, and wherein the pharmaceutical composition does not comprise microcrystalline cellulose.

In another preferred embodiment of the present invention, the pharmaceutical composition consists of omarigliptin or a pharmaceutically acceptable salt thereof, a diluent, a disintegrant and a lubricant, wherein the diluent is maltodextrin, the disintegrant is croscarmellose sodium, and the lubricant is magnesium stearate.

The disintegrant is preferably present in the pharmaceutical composition in an amount of 1 to 15% by weight.

The lubricant is preferably present in the pharmaceutical composition in an amount of 1 to 3% by weight, more preferably in an amount of 2 to 3% by weight.

In another preferred embodiment, the pharmaceutical composition of the present invention may comprise a glidant. The glidant is, for example, silicon dioxide. The glidant can be present in the pharmaceutical composition of the present invention in an amount of 0.2 to 2% by weight, preferably in an amount of 0.5 to 1% by weight.

Omarigliptin can be present in the pharmaceutical composition of the present invention in the form of a free base or in the form of a pharmaceutically acceptable salt thereof. Pharmaceutically acceptable salts of omarigliptin include, without limitation, the hydrochloride, nitrate, phosphate, sulfate, acetate, benzoate, citrate, malate, maleate, mesylate, besylate, succinate, tartrate, oxalate, lactate, fumarate and mandelate salt. Preferred pharmaceutically acceptable salts of omarigliptin are the hydrochloride, nitrate, phosphate, fumarate and mandelate salt. A particularly preferred pharmaceutically acceptable salts of omarigliptin is the fumarate salt. Another particularly preferred pharmaceutically acceptable salt of omarigliptin is the mandelate salt (including the omarigliptin R-mandelate, omarigliptin S-mandelate and omarigliptin RS-mandelate). Another particularly preferred pharmaceutically acceptable salt of omarigliptin is the hydrochloride salt. The salts can be prepared as known to a skilled person or as described in patent application number EP15201150.8.

Omarigliptin or the pharmaceutically acceptable salt thereof is preferably present in the pharmaceutical composition in an amount of 12.5 mg or 25 mg, calculated as a free base.

Omarigliptin can be prepared as described in WO 2010/056708. Omarigliptin exists in several polymorphic forms and in amorphous form. The pharmaceutical composition according to the present invention may comprise omarigliptin in all polymorphic forms or in amorphous form. Preferably, omarigliptin form I or omarigliptin form V is present in the pharmaceutical composition according to the present invention. The polymorphic forms are described, for example, in WO 2013/003249 and in patent application number PCT/EP2016/069687.

Omarigliptin is preferably present in the pharmaceutical composition according to the present invention in an amount of more than 15% by weight, preferably more than 20% by weight, more preferably more than 25% by weight, even more preferably more than 30% by weight, based on the pharmaceutical composition. In particular, omarigliptin is preferably present in the pharmaceutical composition according to the present invention in an amount of more than 15% to less than 50% by weight, preferably more than 20% to less than 50% by weight, more preferably more than 25% to less than 50% by weight, even more preferably more than 30% to less than 50% by weight, based on the pharmaceutical composition.

The pharmaceutical composition according to the present invention is preferably a solid oral dosage form, more preferably a tablet, a capsule or a powder. The tablet may be an uncoated tablet or a coated tablet. The uncoated tablet may be a tablet to be swallowed or a chewable tablet. In a particularly preferred embodiment, the pharmaceutical composition according to the present invention is a coated tablet. The tablet may have a round, oval or biconvex shape.

The coating preferably comprises hydroxypropyl methylcellulose (HPMC), hydroxypropyl cellulose (HPC) and titanium dioxide. The coating may additionally comprise talc. Optionally, a colorant, for example iron oxide red, iron oxide yellow, or an aluminum lake, may be present in the coating.

Preferably, the pharmaceutical composition of the present invention is obtainable by a process comprising a direct compression step.

In particular, the tablets can be manufactured by first mixing omarigliptin or a pharmaceutically acceptable salt thereof with the excipients, except the magnesium stearate, in a suitable mixer. Optionally, the mixture is then passed through a sieve, for example, through a 1.0 mm sieve. Thereafter, the magnesium stearate is added to the mixture. The thus obtained mixture is then blended in a suitable mixer, for example a Turbula mixer, for 1 to 5 minutes, preferably for about 3 minutes, and then compressed into tablets on a suitable tablet press, for example, a Korsch tablet press.

The tablets can be coated in a suitable coating device, for example, a pan coater or a fluid bed coating device.

The capsules can be prepared by mixing omarigliptin or a pharmaceutically acceptable salt thereof with one or more excipients, optionally sieving the mixture, and filling the mixture into capsules, for example into hard gelatine capsules.

The powder can be prepared by mixing omarigliptin or a pharmaceutically acceptable salt thereof with one or more excipients, optionally sieving the mixture, and filling the mixture into sachets.

The pharmaceutical composition of the present invention can be used in the treatment of metabolic diseases, in particular in the treatment of diabetes mellitus type 2.

Within the meaning of this invention, a pharmaceutical composition is "stable" if less than 0.5% by weight of total impurities are formed under accelerated stability test conditions of 50°C/75% relative humidity in a sealed vial or in an open vial after one week.

### EXAMPLES

The following examples illustrate the present invention and are not intended to limit the scope of the invention set forth in the claims appended hereto.

### Example 1

**Tablet formulation**

| **Component** | **Amount (mg)** | **Amount (% by weight)** | **Amount (mg)** | **Amount (% by weight)** |
|---|---|---|---|---|
| Omarigliptin | 25 | 15.63 | 12.5 | 15.63 |
| Maltodextrin | 111 | 69.38 | 55.5 | 69.38 |
| Croscarmellose sodium | 20 | 12.5 | 10 | 12.5 |
| Magnesium stearate | 4 | 2.5 | 2 | 2.5 |
| | | | | |
| Sum components | 160 | 100 | 80 | 100 |
| Sum excipients | 135 | 84.38 | 67.5 | 84.38 |

All components in the amounts mentioned in the above table, except the magnesium stearate, are mixed and passed through a 1.0 mm sieve. The magnesium stearate in the amount mentioned in the above table is added to the mixture. The thus obtained mixture is blended in a Turbula mixer for 3 minutes and then compressed into round tablets having a diameter of 8 mm (tablets containing 25 mg omarigliptin) or 6 mm (tablets containing 12.5 mg omarigliptin) in a Korsch XP1, 8 OR tablet press.

### Example 2

**Tablet formulation**

| **Component** | **Amount (mg)** | **Amount (% by weight)** | **Amount (mg)** | **Amount (% by weight)** |
|---|---|---|---|---|
| Omarigliptin | 25 | 31.02 | 12.5 | 31.02 |
| Maltodextrin | 52 | 64.52 | 26 | 64.52 |
| Croscarmellose sodium | 1.6 | 1.99 | 0.8 | 1.99 |
| Magnesium stearate | 2 | 2.48 | 1 | 2.48 |
| | | | | |
| Sum components | 80.6 | 100 | 40.3 | 100 |
| Sum excipients | 55.6 | 68.98 | 27.8 | 68.98 |

All components in the amounts mentioned in the above table, except the magnesium stearate, are mixed and passed through a 1.0 mm sieve. The magnesium stearate in the amount mentioned in the above table is added to the mixture. The thus obtained mixture is blended in a Turbula mixer for 3 minutes and then compressed into round tablets having a diameter of 6 mm (tablets containing 25 mg omarigliptin) or 4 mm (tablets containing 12.5 mg omarigliptin) in a Korsch XP1, 8 OR tablet press.

### Example 3

**Tablet formulation**

| **Component** | **Amount (mg)** | **Amount (% by weight)** | **Amount (mg)** | **Amount (% by weight)** |
|---|---|---|---|---|
| Omarigliptin | 25 | 15.78 | 12.5 | 15.78 |
| Maltodextrin | 109.4 | 69.07 | 54.7 | 69.07 |
| Croscarmellose sodium | 20 | 12.63 | 10 | 12.63 |
| Magnesium stearate | 4 | 2.53 | 2 | 2.53 |
| | | | | |
| Sum components | 158.4 | 100.00 | 79.2 | 100.00 |
| Sum excipients | 133.4 | 84.22 | 66.7 | 84.22 |

All components in the amounts mentioned in the above table, except the magnesium stearate, are mixed and passed through a 1.0 mm sieve. The magnesium stearate in the amount mentioned in the above table is added to the mixture. The thus obtained mixture is blended in a Turbula mixer for 3 minutes and then compressed into round tablets having a diameter of 8 mm (tablets containing 25 mg omarigliptin) or 6 mm (tablets containing 12.5 mg omarigliptin) in a Korsch XP1, 8 OR tablet press.

### Example 4

**Coated tablet formulation**

| Component | Amount (mg) | Amount (% by weight) | Amount (mg) | Amount (% by weight) |
|---|---|---|---|---|
| Omarigliptin | 25 | 15.63 | 12.5 | 15.63 |
| Maltodextrin | 111 | 69.38 | 55.5 | 69.38 |
| Croscarmellose sodium | 20 | 12.5 | 10 | 12.5 |
| Magnesium stearate | 4 | 2.5 | 2 | 2.5 |
| | | | | |
| Sum components | 160 | 100 | 80 | 100 |
| Sum excipients | 135 | 84.38 | 67.5 | 84.38 |

All components in the amounts mentioned in the above table, except the magnesium stearate, are mixed and passed through a 1.0 mm sieve. The magnesium stearate in the amount mentioned in the above table is added to the mixture. The thus obtained mixture is blended in a Turbula mixer for 3 minutes and then compressed into round tablets having a diameter of 8 mm (tablets containing 25 mg omarigliptin) or 6 mm (tablets containing 12.5 mg omarigliptin) in a Korsch XP1, 8 OR tablet press.

The tablets containing 25 mg omarigliptin are coated in a Glatt Minicoater with an aqueous solution of 6.4 mg of the commercially available coating Opadry® Blue 20A99172 (manufactured by Colorcon) which is made of hydroxypropyl methylcellulose (HPMC), hydroxypropyl cellulose (HPC), titanium dioxide, and FD&C Blue #2/Indigocarmine aluminum lake.

The tablets containing 12.5 mg omarigliptin are coated in a Glatt Minicoater with an aqueous solution of 4.8 mg of the commercially available coating Opadry® Yellow 20A92710 (manufactured by Colorcon) which is made of hydroxypropyl methylcellulose (HPMC), hydroxypropyl cellulose (HPC), titanium dioxide, talc, and iron oxide yellow.

### Example 5

**Coated tablet formulation**

| Component | Amount (mg) | Amount (% by weight) | Amount (mg) | Amount (% by weight) |
|---|---|---|---|---|
| Omarigliptin | 25 | 31.02 | 12.5 | 31.02 |
| Maltodextrin | 52 | 64.52 | 26 | 64.52 |
| Croscarmellose sodium | 1.6 | 1.99 | 0.8 | 1.99 |
| Magnesium stearate | 2 | 2.48 | 1 | 2.48 |
| | | | | |
| Sum components | 80.6 | 100 | 40.3 | 100 |
| Sum excipients | 55.6 | 68.98 | 27.8 | 68.98 |

All components in the amounts mentioned in the above table, except the magnesium stearate, are mixed and passed through a 1.0 mm sieve. The magnesium stearate in the amount mentioned in the above table is added to the mixture. The thus obtained mixture is blended in a Turbula mixer for 3 minutes and then compressed into round tablets having a diameter of 6 mm (tablets containing 25 mg omarigliptin) or 4 mm (tablets containing 12.5 mg omarigliptin) in a Korsch XP1, 8 OR tablet press.

The tablets containing 25 mg omarigliptin are coated in a Glatt Minicoater with an aqueous solution of 4.8 mg of the commercially available coating Opadry® Blue 20A99172 (manufactured by Colorcon) which is made of hydroxypropyl methylcellulose (HPMC), hydroxypropyl cellulose (HPC), titanium dioxide, and FD&C Blue #2/Indigocarmine aluminum lake.

The tablets containing 12.5 mg omarigliptin are coated in a Glatt Minicoater with an aqueous solution of 3.6 mg of the commercially available coating Opadry® Yellow 20A92710 (manufactured by Colorcon) which is made of hydroxypropyl methylcellulose (HPMC), hydroxypropyl cellulose (HPC), titanium dioxide, talc, and iron oxide yellow.

### Example 6

**Coated tablet formulation**

| Component | Amount (mg) | Amount (% by weight) | Amount (mg) | Amount (% by weight) |
|---|---|---|---|---|
| Omarigliptin | 25 | 15.78 | 12.5 | 15.78 |
| Maltodextrin | 109.4 | 69.07 | 54.7 | 69.07 |
| Croscarmellose sodium | 20 | 12.63 | 10 | 12.63 |
| Magnesium stearate | 4 | 2.53 | 2 | 2.53 |
| | | | | |
| Sum components | 158.4 | 100.00 | 79.2 | 100.00 |
| Sum excipients | 133.4 | 84.22 | 66.7 | 84.22 |

All components in the amounts mentioned in the above table, except the magnesium stearate, are mixed and passed through a 1.0 mm sieve. The magnesium stearate in the amount mentioned in the above table is added to the mixture. The thus obtained mixture is blended in a Turbula mixer for 3 minutes and then compressed into round tablets having a diameter of 8 mm (tablets containing 25 mg omarigliptin) or 6 mm (tablets containing 12.5 mg omarigliptin) in a Korsch XP1, 8 OR tablet press.

The tablets containing 25 mg omarigliptin are coated in a Glatt Minicoater with an aqueous solution of 6.4 mg of the commercially available coating Opadry® Blue 20A99172 (manufactured by Colorcon) which is made of hydroxypropyl methylcellulose (HPMC), hydroxypropyl cellulose (HPC), titanium dioxide, and FD&C Blue #2/Indigocarmine aluminum lake.

The tablets containing 12.5 mg omarigliptin are coated in a Glatt Minicoater with an aqueous solution of 4.8 mg of the commercially available coating Opadry® Yellow 20A92710 (manufactured by Colorcon) which is made of hydroxypropyl methylcellulose (HPMC), hydroxypropyl cellulose (HPC), titanium dioxide, talc, and iron oxide yellow.

### Example 7

**Powder formulation**

| Component | Amount (mg) | Amount (% by weight) | Amount (mg) | Amount (% by weight) |
|---|---|---|---|---|
| Omarigliptin | 25 | 8.33 | 12.5 | 4.17 |
| Maltodextrin | 275 | 91.67 | 287.5 | 95.83 |
| | | | | |
| Sum components | 300 | 100.00 | 300 | 100.00 |
| Sum excipients | 275 | 91.67 | 287.5 | 95.83 |

All components in the amounts mentioned in the above table are mixed and passed through a 1.0 mm sieve. The thus obtained mixture is put into sachets.

### Example 8

**Capsule formulation**

| Component | Amount (mg) | Amount (% by weight) | Amount (mg) | Amount (% by weight) |
|---|---|---|---|---|
| Omarigliptin | 25 | 29.24 | 12.5 | 29.24 |
| Maltodextrin | 60 | 70.18 | 30 | 70.18 |
| Silicon dioxide | 0.5 | 0.58 | 0.25 | 0.58 |
| | | | | |
| Sum components | 85.50 | 100.00 | 42.75 | 100.00 |
| Sum excipients | 60.5 | 70.76 | 30.25 | 70.76 |

All components in the amounts mentioned in the above table are mixed, passed through a 1.0 mm sieve and filled into hard gelatine capsules.

## Claims

1. A pharmaceutical composition comprising
omarigliptin or a pharmaceutically acceptable salt thereof, and
a diluent,
wherein the diluent comprises a reducing sugar, and
wherein the reducing sugar is maltodextrin.

2. Pharmaceutical composition according to claim 1, wherein the maltodextrin has a dextrose equivalent of 1 to 20, preferably 3 to 20, more preferably 5 to 10.

3. Pharmaceutical composition according to claim 1 or 2, wherein the maltodextrin is the only reducing sugar present in the pharmaceutical composition.

4. Pharmaceutical composition according to any of claims 1 to 3, wherein the maltodextrin is the only diluent present in the pharmaceutical composition.

5. Pharmaceutical composition according to any of claims 1 to 4, wherein the pharmaceutical composition is free of mannitol.

6. Pharmaceutical composition according to any of claims 1 to 5, wherein the pharmaceutical composition is free of microcrystalline cellulose.

7. Pharmaceutical composition according to any of claims 1 to 6, wherein the pharmaceutical composition is free of mannitol and free of microcrystalline cellulose.

8. Pharmaceutical composition according to any of claims 1 to 7, wherein the maltodextrin is present in the pharmaceutical composition in an amount of 60 to 99% by weight, preferably 60 to 90% by weight, more preferably 60 to 80% by weight, even more preferably 60 to 75% by weight, based on the pharmaceutical composition.

9. Pharmaceutical composition according to any of claims 1 to 8 comprising additionally a disintegrant and/or a lubricant.

10. Pharmaceutical composition according to claim 9, wherein the disintegrant is croscarmellose sodium.

11. Pharmaceutical composition according to claim 9, wherein the lubricant is magnesium stearate.

12. Pharmaceutical composition according to any of claims 9 to 11, wherein the disintegrant is croscarmellose sodium and the lubricant is magnesium stearate.

13. Pharmaceutical composition according to any of claims 1 to 12 which is a solid oral dosage form, preferably a tablet, a capsule or a powder.

14. Pharmaceutical composition according to claim 13 which is a tablet, preferably an uncoated tablet or a coated tablet.

15. Pharmaceutical composition according to any of claims 1 to 14, wherein omarigliptin or a pharmaceutically acceptable salt thereof is present in an amount of 12.5 mg or 25 mg, calculated as a free base.
